# EUROPÄISCHE PATENTANMELDUNG

(11) **EP 2 537 919 A1**
(43) Veröffentlichungstag der Anmeldung: **26.12.2012**
(21) Anmeldenummer: 11005135.6
(22) Anmeldetag: 24.06.2011
(51) Int. Cl.: C12M 1/107

(54) **Anlage zur Erzeugung von Biogas und Verfahren zum Betrieb einer solchen**

(71) Anmelder: KOMPOFERM GmbH, 33428 Marienfeld (DE)
(72) Erfinder: Eggersmann, Karlgünter, 33428 Marienfeld (DE)
(74) Vertreter: Schober, Mirko

(57) **Zusammenfassung**

Eine Anlage zur Erzeugung von Biogas weist mindestens einen Fermenter 1 zur Fermentierung feststoffförmiger Biomasse 13 auf. Der Fermenter 1 weist eine Vorrichtung zur Beaufschlagung der Biomasse mit einem flüssigen Medium, einen Gasauslass 2 zur Abfuhr des Biogases aus dem Fermenter 1 und einen Gaseinlass 3 zur Zufuhr von Gasen in den Fermenter 1 auf. Der Gaseinlass 3 ist mit einer Biogasquelle, vorzugsweise mit dem Gasauslass 2, einer Biogasleitung und/oder einem Biogasspeicher verbindbar.

## Beschreibung

Die Erfindung betrifft eine Anlage zur Erzeugung von Biogas nach dem Oberbegriff des Anspruchs 1 sowie ein Verfahren zum Betrieb einer Anlage zur Erzeugung von Biogas nach dem Oberbegriff des Anspruchs 9.

Unter Biogas wird dabei ein methanreiches Gas verstanden, welches bei der anaeroben Vergaerung von Biomasse entsteht. Die anaerobe Vergärung wird in diesem Zusammenhang auch als Fermentierung bzw. Fermentation bezeichnet. Nach dem Stand der Technik bekannte Anlagen zur Fermentierung feststoffförmiger Biomasse weisen im Wesentlichen einen in der Regel gasdichten Fermenter zur Aufnahme der feststoffförmigen Biomasse auf. Dabei kann es sich um ein garagen- oder tunnelförmiges Bauwerk handeln, in welches die Biomasse beispielsweise in Form eines Haufwerks eingebracht wird.

Fermenter nach dem Stand der Technik weisen darüber hinaus in der Regel eine Vorrichtung zur Beaufschlagung der Biomasse mit einem flüssigen Medium auf. Bei dem flüssigen Medium handelt es sich in der Regel um sogenanntes Perkolat, das durch die Biomasse, welche normalerweise in Form einer Schüttung oder als Haufwerk vorliegt und somit eine gewisse Porosität aufweist, hindurchgeleitet wird. Das Perkolat dient dabei insbesondere auch als Träger für die anaerobe Biologie, die für den Fermentationsprozess verantwortlich ist und ermöglicht in der Regel erst die Fermentierung feststoffförmiger Biomasse, die durch das Perkolat benetzt und damit befeuchtet wird. Da die Biomasse dabei nicht wie bei einer klassischen Nassfermentation angemaischt wird, sondern ihre Haufwerksstruktur zumindest zunächst weitgehend beibehält, wird bei derartigen Prozessen auch von Trockenfermentation gesprochen, auch wenn die Fermentation selber streng genommen in einem feuchten, nämlich durch das Perkolat benetzten Milieu stattfindet.

Die Fermenter der in Rede stehenden Art weisen in der Regel einen Gasauslass zur Abfuhr des Biogases aus dem Fermenter sowie einen Gaseinlass zur Zufuhr von Gasen in den Fermenter auf. Dabei dient der Gasauslass zumindest primär zur Abfuhr des Biogases aus dem Fermenter, während der Gaseinlass in der Regel dazu verwendet wird, den Fermenter zu spülen, beispielsweise um mit einem kohlendioxidhaltigen Gas den Sauerstoff aus dem Fermenter zu verdrängen, um beim Übergang von aerober zu anaerober Phase unter anderem die Explosionsgefahr zu verringern, indem explosionsfähige Gasgemische oder Gase, die zur Bildung solcher Gemische führen können, abgeführt werden.

Derartige Spülvorgänge, die insbesondere beim An- und Abfahren des Fermenters und dem damit verbundenen Umschalten zwischen aeroben und anaeroben Prozessen vorgenommen werden müssen, bringen das Problem mit sich, dass während des Spülens eine große Menge Gas mit einer technisch nicht mehr sinnvoll nutzbaren Zusammensetzung gebildet wird, die in der Regel einer Entsorgung beispielsweise in speziellen Fackeln oder Filteranlagen zugeführt werden muss. Insbesondere aufgrund der Porosität des Feststoffs in dem Fermenter geht auf diese Weise eine beträchtliche Menge an Biogas verloren, was den Prozess ineffizient gestaltet.

Der Erfindung liegt die Aufgabe zugrunde, eine Anlage zur Erzeugung von Biogas und ein Verfahren zum Betrieb einer Anlage zur Erzeugung von Biogas anzugeben, welche eine effizientere Biogaserzeugung insbesondere im Hinblick auf die Vermeidung von Gasverlusten durch Spülprozesse und einem höheren möglichen Gehalt an Trockensubstrat ermöglichen.

Die Aufgabe wird gelöst durch eine Anlage mit den Merkmalen des Anspruchs 1 und ein Verfahren mit den Merkmalen des Anspruchs 9. Vorteilhafte Ausführungsformen finden sich in den Unteransprüchen.

Erfindungsgemäß wird die Aufgabe dadurch gelöst, dass Biogas in den Fermenter eingeleitet wird. Zu diesem Zweck ist erfindungsgemäß der Gaseinlass des Fermenters mit einer Biogasquelle, vorzugsweise dem Gasauslass des gleichen und/oder eines anderen Fermenters, einer Biogasleitung oder einem Biogasspeicher verbindbar. Dies ist in der Regel durch Leitungen und Absperrarmaturen realisiert, mit denen die zum jeweiligen Prozessschritt notwendigen Leitungsverbindungen nach Bedarf hergestellt und getrennt werden können. Bei Anlagen, die eine Mehrzahl Fermenter aufweisen, ist es dabei vorteilhaft, die Gasauslässe der verschiedenen Fermenter mit einer gemeinsamen Biogasleitung verbindbar zu gestalten. Das zur Einleitung in einen Fermenter bestimmte Biogas kann dann vorteilhafterweise der gemeinsamen Biogasleitung entnommen werden, was den Vorteil mit sich bringt, dass die Biogasversorgung über die gemeinsam genutzte Biogasleitung zuverlässiger sichergestellt werden kann, als bei der Entnahme aus einem einzelnen Fermenter. Insbesondere dann, wenn die an der gemeinsamen Biogasleitung betriebenen Fermenter derart betrieben werden, dass die verschiedenen Prozessschritte des Fermenterbetriebs, insbesondere An- und Abfahrbetrieb, in verschiedenen Fermentern zeitversetzt durchgeführt werden, so dass im angestrebten Fall immer mindestens ein Fermenter sich in einer Biogas erzeugenden Phase befindet.

Dabei ist es vorteilhaft, wenn der oder die Fermenter über einen weiteren Gasauslass neben dem Biogasauslass verfügen bzw. der Biogasauslass über eine schaltbare bzw. absperrbare Abzweigung verfügt, die zu einer geeigneten Gasentsorgungseinrichtung, beispielsweise einer Fackel, insbesondere Schwachgasfackel, oder einem Biofilter oder einer anderen Abluftbehandlung führt. Fallen bei An- oder Abfahrvorgängen bzw. Spülprozessen Gase an, die nicht zur Verwertung dem Biogassystem zugeführt werden können, so können diese über die weiteren Gasauslässe bzw. Abzweigungen der Biogasleitungen entsorgt und/oder entsprechend behandelt werden.

Vorzugsweise weist die Anlage dabei eine Fördereinrichtung zur Förderung des Biogases in Richtung des Gaseinlasses auf. Bei dieser Fördereinrichtung kann es sich beispielsweise um einen Kompressor bzw. Verdichter handeln, der in der Lage ist, das Biogas in Richtung des Fermenters zu fördern. Vorteil dabei ist zum einen, dass die korrekten Strömungsrichtungen im Biogasleitungssystem hiermit sichergestellt werden können, zum anderen kann das Biogas auch gegen Druckwiderstände in den Fermenter gefördert werden, was insbesondere dann vorteilhaft ist, wenn das Biogas derart in den Fermenter geleitet wird, dass eine Durchströmung der Biomasse durch das Biogas erzwungen wird.

Wie bereits oben angeführt, weist das Biomassehaufwerk bzw. die Biomasseschüttung eine innere Porosität bzw. Kavitäten im Inneren auf, in denen sich Gas sammelt, insbesondere kann es sich dabei um Lufteinschlüsse handeln, die nach dem Einbringen der Biomasse in den Fermenter in der Biomasse vorhanden sind. Um diese Lufteinschlüsse möglichst effizient entfernen zu können, ist es sinnvoll, dass das Biogas derart in den Fermenter geleitet wird, dass eine Durchströmung der Biomasse durch das Biogas erzwungen wird. Ein weiterer Vorteil dieser Vorgehensweise ist, dass auf diese Weise Drainagekanäle im Haufwerk geschaffen werden können. Zu diesem Zweck ist es insbesondere vorteilhaft, das Biogas auch während der Fermentationsphase, d.h. während der Zufuhr des flüssigen Mediums in den Fermenter und der Beaufschlagung der feststoffförmigen Biomasse mit dem flüssigen Medium, Biogas in den Fermenter einzuleiten. Die Schaffung der Drainagekanäle ist in dem Fall vorteilhaft, auch wenn bei der Biogaseinleitung in dieser Phase des Prozesses durch das Biogas kein anderes Gas verdrängt wird. Insbesondere in dieser Phase kann es auch sinnvoll sein, das Biogas eines einzigen Fermenters im Kreislauf zu fahren.

Diese erzwungene Durchströmung der Biomasse erfolgt vorzugsweise ausgehend vom Boden des Fermenters in überwiegend vertikaler Richtung. Auf diese Weise kann insbesondere dann, wenn der Gaseinlass als Gasverteileinrichtung, insbesondere als Spigotboden, ausgeführt ist, ein Drainagekanalnetzwerk im Haufwerk bzw. der Schüttung der feststoffförmigen Biomasse geschaffen werden, welches eine möglichst homogene Durchströmung der feststoffförmigen Biomasse mit dem flüssigen Medium ermöglicht. Alternativ besteht auch die Möglichkeit, das Spülgas seitlich in das Haufwerk einzuleiten. Unabhängig von der Durchströmungsrichtung ist die bessere Entwässerung des Haufwerks ein weiterer Vorteil.

Der Gaseinlass ist daher vorzugsweise unterhalb der in den Fermenter einzubringenden Biomasse angeordnet, vorzugsweise im Boden des Fermenters. Dies ist insbesondere dann sinnvoll, wenn innerhalb des Fermenters eine, vorzugsweise für flüssige Medien durchlässige, Einrichtung zur seitlichen Abstützung der feststoffförmigen Biomasse vorgesehen ist. Das Vorsehen einer derartigen Abstützung ist vorteilhaft, um eine hohe Haufwerkshöhe zu ermöglichen und gleichzeitig das Abfließen des flüssigen Mediums seitlich aus dem Haufwerk bzw. der Schüttung der Biomasse zu ermöglichen. Diese Bauweise ist jedoch anfällig für das Entstehen eines Totvolumens im unteren mittigen Bereich des Haufwerks, welches unbefriedigend vom flüssigen Medium durchströmt wird, was wirkungsvoll durch die vorteilhafte Anordnung des Gaseinlasses unterhalb der Biomasse verhindert werden kann. Der dann vorteilhafterweise auch ein Abführen des flüssigen Mediums nach unten ermöglicht.

Besonders vorteilhaft kann das Biogas nach der Beendigung der Zufuhr des flüssigen Mediums zur Unterstützung der Entwässerung der Biomasse in den Fermenter geleitet werden. Dadurch ist es möglich, den Fermentationsprozess bis zum Erreichen des für die Entnahme der Biomasse aus dem Fermenter angestrebten Wasser- bzw. Trockensubstratgehalts aufrecht zu erhalten. Zwischen der Fermentationsphase unter Biogasatmosphäre zur Erzeugung des Biogases und der Entnahme der fermentierten Biomasse, die zwangsweise mit einer Belüftung des Fermenters einhergeht, ist so nur ein minimaler kurzer Spülvorgang des Fermenters notwendig.

Ebenfalls vorteilhaft ist es, das Biogas zur Spülung des Fermenters während des Anfahrens des Fermenters vorteilhafterweise zur Verdrängung von Gasen, insbesondere Sauerstoff und Stickstoff, aus dem Fermenter und insbesondere aus den Porositäten und/oder Kavitäten der Biomasse in dem Fermenter einzusetzen. Dabei kann bereits nach einem kurzen Spülvorgang mit der anaeroben Fermentation begonnen und nutzbares Biogas erzeugt werden. Die Entstehung von technisch nicht verwertbaren Gasgemischen während des Anfahrbetriebs wird dabei vermieden. Zur Realisierung der Spülung bei An- und Abfahrvorgängen ist es insbesondere vorteilhaft, wenn die Anlage über eine Mehrzahl Fermenter verfügt und Gaseinlass und Gasauslass verschiedener Fermenter miteinander verbindbar sind. Dies kann beispielsweise über die Ver-schaltung mit einer gemeinsamen Biogasleitung geschehen. Vorzugsweise kann dabei auch ein Biogasspeicher vorgesehen sein, der vorzugsweise mit Gasauslass und/oder Gaseinlass vorzugsweise sämtlicher vorhandener Fermenter verbindbar ist und zur Sicherstellung der Verfügbarkeit des Biogases dient.

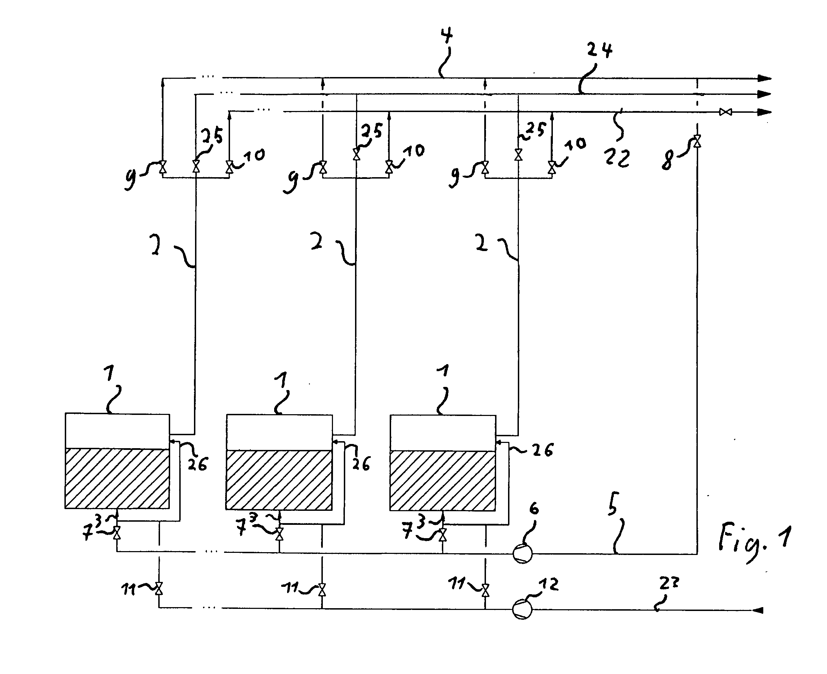

Die Erfindung wird im Folgenden anhand der Figuren 1 bis 4 schematisch näher erläutert:
- Figur 1 -: zeigt ein Fließbild einer beispielhaften erfindungsgemäßen Anlage,
- Figur 2 -: zeigt schematisch die Strömungswege des Perkolats und die Entstehung eines Totvolumens ohne erfindungsgemäße Biogaszufuhr,
- Figur 3 -: zeigt schematisch die Strömungswege zugeführten Biogases durch die Biomasse in einem beispielhaften erfindungsgemäßen Fermenter,
- Figur 4 -: zeigt schematisch beispielhafte Strömungswege des flüssigen Mediums durch die Biomasse nach Schaffung zusätzlicher Drainagekanäle mit einem beispielhaften erfindungsgemäßen, vorteilhaften Spülverfahren.

Die beispielhafte Anlage weist eine Mehrzahl Fermenter 1 auf, deren Gasauslässe 2 über die Absperrarmaturen 9 mit der gemeinsamen Biogasleitung 4 verbunden werden können. Weiterhin ist es möglich, die Gasauslässe 2 mit der zusätzlichen Abgasleitung 22 mittels der Absperrarmaturen 10 zu verbinden. Die beispielhafte Anlage verfügt darüber hinaus über eine zusätzliche Schutzgasleitung 24, die mittels der Absperrarmaturen 25 mit den Fermentern 1 verbunden werden kann.

Über die Absperrarmatur 8 und die Leitung 5 sind die Gaseinlässe 3 der einzelnen Fermenter mit der gemeinsamen Biogasleitung 4 verbindbar. Dabei können die Gaseinlässe 3 einzeln mit ihren jeweiligen Absperrarmaturen 7 mit der Leitung 5 verbunden oder von dieser getrennt werden. In der Leitung 5 ist eine Fördereinrichtung 6, beispielsweise ein Verdichter, vorgesehen, um die Förderung des Biogases zu den Gaseinlässen sicherzustellen. Zusätzlich verfügt die erfindungsgemäße Anlage über eine weitere Spülgaszufuhrleitung 23, mit der beispielsweise Luft zu den Gaseinlässen in den Böden der Fermenter 1 und/oder weiteren Gaseinlässen 26 im Bereich der Kopfräume der Fermenter 1 gefördert werden kann. Zu diesem Zweck ist eine weitere Fördereinrichtung 12 vorgesehen. Die einzelnen Gaseinlässe 3 können durch die Absperrarmaturen 11 mit dieser Leitung verbunden werden.

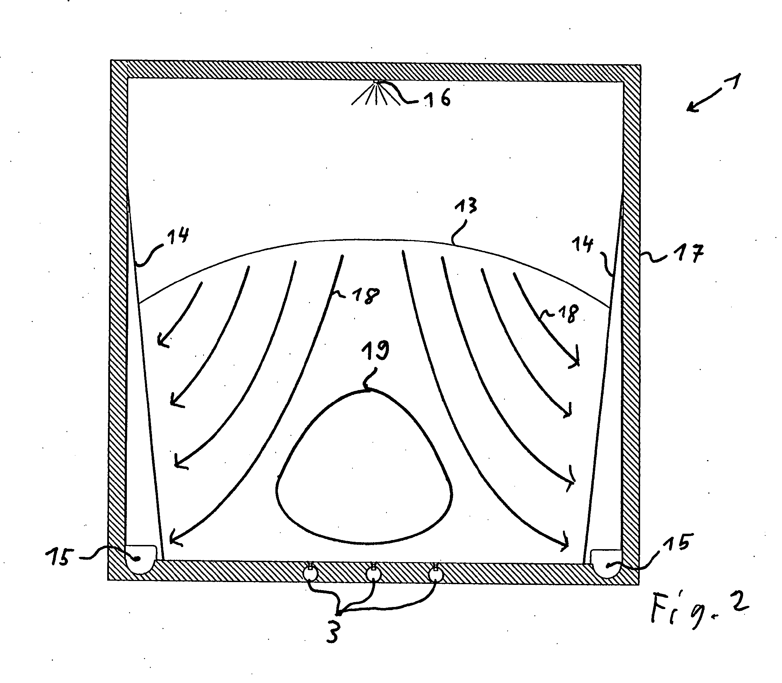

Die beispielhaften erfindungsgemäßen Fermenter 1 weisen in ihrem als Spigotboden ausgeführten Boden Gaseinlässe 3 auf. Innerhalb der gasdichten Hülle 17 des Fermenters ist weiterhin eine Vorrichtung 16 zur Beaufschlagung der Biomasse 13 mit einem flüssigen Medium angeordnet. Die Biomasse 13 im Fall der beispielhaften Anlage wird seitlich durch die Einrichtung 14, die für das flüssige Medium durchlässig ist, abgestützt. Der Behälter weist im Fall der beispielhaften Anlage weiterhin Sammelkanäle 15 zur Abfuhr des flüssigen Mediums auf. Ohne den Einsatz der erfindungsgemäßen Spülung zur Schaffung von zusätzlichen Drainagekanälen im Haufwerk der Biomasse 13, nimmt das flüssige Medium beim Durchdringen des Haufwerks die in Figur 2 angedeuteten bevorzugten Strömungswege 18. Hierdurch entsteht im unteren mittleren Bereich des Haufwerks der Biomasse 13 ein Totvolumen 19, welches nicht hinreichend mit dem flüssigen Medium durchströmt wird.

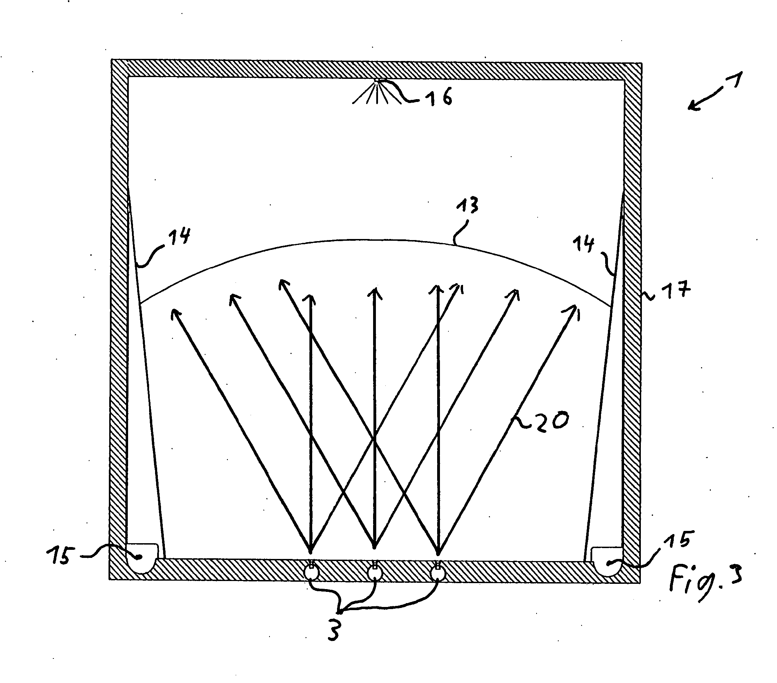

Wird durch den Gaseinlass 3 Biogas in den Fermenter 1 eingeleitet, so wird das Haufwerk der Biomasse 13 erzwungenermaßen von dem eingeleiteten Biogas durchströmt, wobei aufgrund der Anordnung des Gaseinlasses 3 im Boden des Fermenters eine vorwiegend vertikale Durchströmung der Biomasse 13 entsteht. Die vorherrschenden Strömungswege und Richtungen 20 sind dabei in Figur 3 schematisch dargestellt.

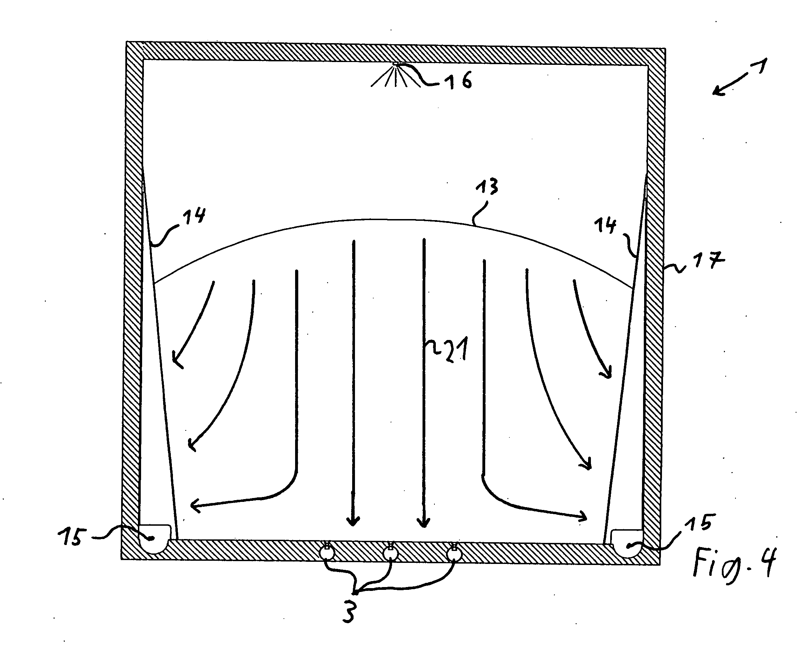

Durch die so geschaffenen zusätzlichen Drainagekanäle im Haufwerk der Biomasse 13 wird so eine homogenere Durchströmung der Biomasse 13 und eine Unterdrückung der Bildung von Totvolumina erzielt, wie durch die schematisch dargestellten Strömungswege 21 in Figur 4 dargestellt. Das flüssige Medium wird nach dem Passieren der Biomasse 13 über die Sammelkanäle 15 und/oder die Gaseinlässe 3 abgeführt.

## Patentansprüche

1. Anlage zur Erzeugung von Biogas, die mindestens einen Fermenter (1) zur Fermentierung feststoffförmiger Biomasse (13) aufweist, wobei der Fermenter (1) eine Vorrichtung zur Beaufschlagung der Biomasse mit einem flüssigen Medium, einen Gasauslass (2) zur Abfuhr des Biogases aus dem Fermenter (1) und einen Gaseinlass (3) zur Zufuhr von Gasen in den Fermenter (1) aufweist,
**dadurch gekennzeichnet,**
**dass** der Gaseinlass (3) mit einer Biogasquelle, vorzugsweise mit dem Gasauslass (2), einer Biogasleitung und/oder einem Biogasspeicher verbindbar ist.

2. Anlage nach Anspruch 1,
**dadurch gekennzeichnet,**
**dass** der Gaseinlass (3) als Gasverteilereinrichtung, insbesondere als Spigotboden, ausgeführt ist.

3. Anlage nach Anspruch 1 oder 2,
**dadurch gekennzeichnet,**
**dass** die Anlage eine Fördereinrichtung (6) zur Förderung des Biogases in Richtung des Gaseinlass (3) aufweist.

4. Anlage nach einem der vorigen Ansprüche,
**dadurch gekennzeichnet,**
**dass** die Anlage einen Biogasspeicher aufweist, der bevorzugt mit dem Gasauslass (2) und/oder mit dem Gaseinlass (3) verbindbar ist.

5. Anlage nach einem der vorigen Ansprüche,
**dadurch gekennzeichnet,**
**dass** innerhalb des Fermenters eine, vorzugsweise für flüssige Medien durchlässige, Einrichtung (14) zur seitlichen Abstützung der Biomasse (13) vorgesehen ist.

6. Anlage nach einem der vorigen Ansprüche,
**dadurch gekennzeichnet,**
**dass** der Gaseinlass (3) unterhalb der in den Fermenter (1) einzubringenden Biomasse angeordnet ist, vorzugsweise im Boden

7. Anlage nach einem der vorigen Ansprüche,
**dadurch gekennzeichnet,**
**dass** die Anlage eine Mehrzahl Fermenter (1) aufweist und Gaseinlass (3) und Gasauslass (2) verschiedener Fermenter (1) miteinander verbindbar sind.

8. Anlage nach Anspruch 7,
**dadurch gekennzeichnet,**
**dass** eine Mehrzahl Gasauslässe (2) verschiedener Fermenter (1) mit einer gemeinsamen Biogasleitung (4) verbindbar ist.

9. Verfahren zum Betrieb einer Anlage zur Erzeugung von Biogas, insbesondere einer Anlage nach Anspruch 1, bei dem feststoffförmige Biomasse (13) in einen Fermenter (1) eingebracht, mit einem flüssigen Medium beaufschlagt und zur Biogaserzeugung anaerob fermentiert wird,
**dadurch gekennzeichnet,**
**dass** Biogas in den Fermenter (1) eingeleitet wird.

10. Verfahren nach Anspruch 9,
**dadurch gekennzeichnet,**
**dass** das Biogas nach Beendigung der Zufuhr des flüssigen Mediums zur Unterstützung der Trocknung der Biomasse in den Fermenter (1) geleitet wird.

11. Verfahren nach Anspruch 9 oder 10,
**dadurch gekennzeichnet,**
**dass** das Biogas während der Zufuhr des flüssigen Mediums zur Schaffung von Drainagekanälen für das flüssige Medium in den Fermenter (1) geleitet wird.

12. Verfahren nach einem der Ansprüche 9 bis 11,
**dadurch gekennzeichnet,**
**dass** das Biogas beim Anfahren des Fermenters (1) zur Verdrängung von Gasen, insbesondere Sauerstoff und Stickstoff, aus dem Fermenter (1), insbesondere aus Porositäten und/oder Kavitäten der Biomasse (13), in den Fermenter (1) geleitet wird.

13. Verfahren nach einem der Ansprüche 9 bis 12,
**dadurch gekennzeichnet,**
**dass** das Biogas derart in den Fermenter geleitet wird,
**dass** eine Durchströmung der Biomasse (13) durch das Biogas erzwungen wird.

14. Verfahren nach Anspruch 13,
**dadurch gekennzeichnet,**
**dass** die erzwungene Durchströmung der Biomasse (13) ausgehend vom Boden des Fermenters (1) in überwiegend vertikaler Richtung erfolgt.

15. Verfahren nach einem der Ansprüche 9 bis 14,
**dadurch gekennzeichnet,**
**dass** das Biogas aus einem anderen Fermenter (1) einer gemeinsamen Biogasleitung (4) mehrerer Fermenter (1) oder einem Bioasspeicher als dem, in den es eingeleitet wird, entnommen wird.

## Geänderte Patentansprüche

### Geänderte Patentansprüche gemäss Regel 137(2) EPÜ.

**1.** Anlage zur Erzeugung von Biogas, die mindestens einen Fermenter (1) zur Fermentierung feststoffförmiger Biomasse (13) aufweist, wobei der Fermenter (1) eine Vorrichtung zur Beaufschlagung der Biomasse (13) mit einem flüssigen Medium, einen Gasauslass (2) zur Abfuhr des Biogases aus dem Fermenter (1) und einen Gaseinlass (3) zur Zufuhr von Gasen in den Fermenter (1) aufweist, wobei der Gaseinlass (3) mit einer Biogasquelle, vorzugsweise mit dem Gasauslass (2), einer Biogasleitung und/oder einem Biogasspeicher verbindbar ist,
**dadurch gekennzeichnet,**
**dass** der Gaseinlass (3) so ausgebildet ist, dass über ihn das flüssige Medium nach dem Passieren der Biomasse abgeführt wird, und dass während der Zufuhr des flüssigen Mediums in den Fermenter (1) Biogas in den Fermenter eingeleitet wird.

**2.** Anlage nach Anspruch 1,
**dadurch gekennzeichnet,**
**dass** der Gaseinlass (3) als Gasverteilereinrichtung, insbesondere als Spigotboden, ausgeführt ist.

**3.** Anlage nach Anspruch 1 oder 2,
**dadurch gekennzeichnet,**
**dass** die Anlage eine Fördereinrichtung (6) zur Förderung des Biogases in Richtung des Gaseinlass (3) aufweist.

**4.** Anlage nach einem der vorigen Ansprüche,
**dadurch gekennzeichnet,**
**dass** die Anlage einen Biogasspeicher aufweist, der bevorzugt mit dem Gasauslass (2) und/oder mit dem Gaseinlass (3) verbindbar ist.

**5.** Anlage nach einem der vorigen Ansprüche,
**dadurch gekennzeichnet,**
**dass** innerhalb des Fermenters eine, vorzugsweise für flüssige Medien durchlässige, Einrichtung (14) zur seitlichen Abstützung der Biomasse (13) vorgesehen ist.

**6.** Anlage nach einem der vorigen Ansprüche,
**dadurch gekennzeichnet,**
**dass** der Gaseinlass (3) unterhalb der in den Fermenter (1) einzubringenden Biomasse angeordnet ist, vorzugsweise im Boden

**7.** Anlage nach einem der vorigen Ansprüche,
**dadurch gekennzeichnet,**
**dass** die Anlage eine Mehrzahl Fermenter (1) aufweist und Gaseinlass (3) und Gasauslass (2) verschiedener Fermenter (1) miteinander verbindbar sind.

**8.** Anlage nach Anspruch 7,
**dadurch gekennzeichnet,**
**dass** eine Mehrzahl Gasauslässe (2) verschiedener Fermenter (1) mit einer gemeinsamen Biogasleitung (4) verbindbar ist.

**9.** Verfahren zum Betrieb einer Anlage zur Erzeugung von Biogas, insbesondere einer Anlage nach Anspruch 1, bei dem feststoffförmige Biomasse (13) in einen Fermenter (1) eingebracht, mit einem flüssigen Medium beaufschlagt und zur Biogaserzeugung anaerob fermentiert wird, wobei Biogas in den Fermenter (1) eingeleitet wird
**dadurch gekennzeichnet,**
**dass** das Biogas während der Zufuhr des flüssigen Mediums durch Gaseinlässe (3) in den Fermenter (1) geleitet wird, so dass **dadurch** Drainagekanäle für das flüssige Medium geschaffen werden, wobei das flüssige Medium nach dem Passieren der Biomasse (13) über die Gaseinlässe (3) abgeführt wird.

**10.** Verfahren nach Anspruch 9,
**dadurch gekennzeichnet,**
**dass** das Biogas nach Beendigung der Zufuhr des flüssigen Mediums zur Unterstützung der Trocknung der Biomasse in den Fermenter (1) geleitet wird.

**11.** Verfahren nach einem der Ansprüche 9 oder 10,
**dadurch gekennzeichnet,**
**dass** das Biogas beim Anfahren des Fermenters (1) zur Verdrängung von Gasen, insbesondere Sauerstoff und Stickstoff, aus dem Fermenter (1), insbesondere aus Porositäten und/oder Kavitäten der Biomasse (13), in den Fermenter (1) geleitet wird.

**12.** Verfahren nach einem der Ansprüche 9 bis 11,
**dadurch gekennzeichnet,**
**dass** das Biogas derart in den Fermenter geleitet wird, dass eine Durchströmung der Biomasse (13) durch das Biogas erzwungen wird.

**13.** Verfahren nach Anspruch 12,
**dadurch gekennzeichnet,**
**dass** die erzwungene Durchströmung der Biomasse (13) ausgehend vom Boden des Fermenters (1) in überwiegend vertikaler Richtung erfolgt.

**14.** Verfahren nach einem der Ansprüche 9 bis 13,
**dadurch gekennzeichnet,**
**dass** das Biogas aus einem anderen Fermenter (1) einer gemeinsamen Biogasleitung (4) mehrerer Fermenter (1) oder einem Bioasspeicher als dem, in den es eingeleitet wird, entnommen wird.
